# EUROPEAN PATENT APPLICATION

(11) **EP 1 580 681 A2**
(43) Date of publication of application: **28.09.2005**
(21) Application number: 05003419.8
(22) Date of filing: 17.02.2005
(51) Int. Cl.: G06F 19/00

(54) **Method for processing missing values in measured data**

(30) Priority: 19.02.2004 KR 2004011001
(71) Applicant: SAMSUNG ELECTRONICS CO., LTD., Gyeonggi-do (KR)
(72) Inventor: Nam, Yun-sun, Seongnam-si Gyeonggi-do (KR); Park, Kyung-hee, Gangnam-gu Seoul (KR)
(74) Representative: Grünecker, Kinkeldey, Stockmair & Schwanhäusser Anwaltssozietät

(57) **Abstract**

A method for processing missing values in measured data is provided. The method for processing missing values in measured data including a plurality of missing values, includes: assigning different weights to measured objects or measured items with respect to the importance of the measured objects or measured items; selecting a set minimizing sum of the weights, among sets of the measured objects or measured items including the missing values; and removing measured objects and measured items included in the selected set, from the measured data.

## Description

The present invention relates to a method for processing missing values, and more particularly, to a method for processing missing values included in genetic data.

A high-throughput genotyping technology has enabled genotyping of a plurality of genes with a large number of samples at the same time. This genotyping technology is used to find a nucleotide sequence of a predetermined sample or single nucleotide polymorphism (SNP) information of a predetermined gene part. Also, the high-throughput genotyping technology is used usefully biologically to search genes related to attacks of diseases or draws genetic maps based on the SNP information. The result of these experiments is expressed in a form of a matrix with samples in rows and genes or SNP positions in columns. Due to the characteristics of the high-throughput genotyping technology, however, it cannot be avoided that a number of values are often missing.

Accordingly, with regard to missing values frequently occurring when genetic experiments are performed on a large scale, a method for processing missing values while minimizing loss in the remaining measured data is needed.

It is the object of the present invention to provide a method by which a different weight is assigned to a measured object or a measured item according to an importance of the measured object or item, a priority of the measured object or measured item is determined according to the weight, and based on the priority, missing values are processed.

This object is solved by the subject matter of the independent claims.

Preferred embodiments are defined by the subject matter of the dependent claims.

According to an aspect of the present invention, there is provided a method for processing missing values in measured data including a plurality of missing values, including: assigning different weights to measured objects or measured items with respect to the importance of the measured objects or measured items; selecting a set minimizing sum of the weights, among sets of the measured objects or measured items including the missing values; and removing measured objects and measured items included in the selected set, from the measured data.

According to another aspect of the present invention, there is provided a method for processing missing values including: assigning different weights to samples or single nucleotide polymorphism positions with respect to the importance of the samples or single nucleotide polymorphism positions in genetic data; selecting a set minimizing a sum of weights, among sets of the samples and single nucleotide polymorphism positions including missing values; and removing samples and single nucleotide polymorphism positions included in the selected set, from the genetic data.

The above and other features and advantages of the present invention will become more apparent by describing in detail exemplary embodiments thereof with reference to the attached drawings in which:
FIG. 1 is a flowchart of a method for processing missing values according to an embodiment of the present invention;
FIG. 2 illustrates missing values occurred in relation to 6 samples and 6 single nucleotide polymorphism (SNP) positions;
FIGS. 3A and 3B show different examples of sets of samples and SNP positions that can be removed in relation to missing values shown in FIG. 2;
FIG. 4 is a bipartite graph of missing values with samples and SNP positions as vertexes;
FIG. 5 illustrates the output of genetic data in which the number of SNP positions is 10, the number of samples is 20, and the number of missing values is 33;
FIG. 6 illustrates examples of weights assigned according to SNP positions; and
FIG. 7 is a bipartite graph of missing values shown in FIG. 5.

The present invention will now be described more fully with reference to the accompanying drawings, in which exemplary embodiments of the invention are shown.

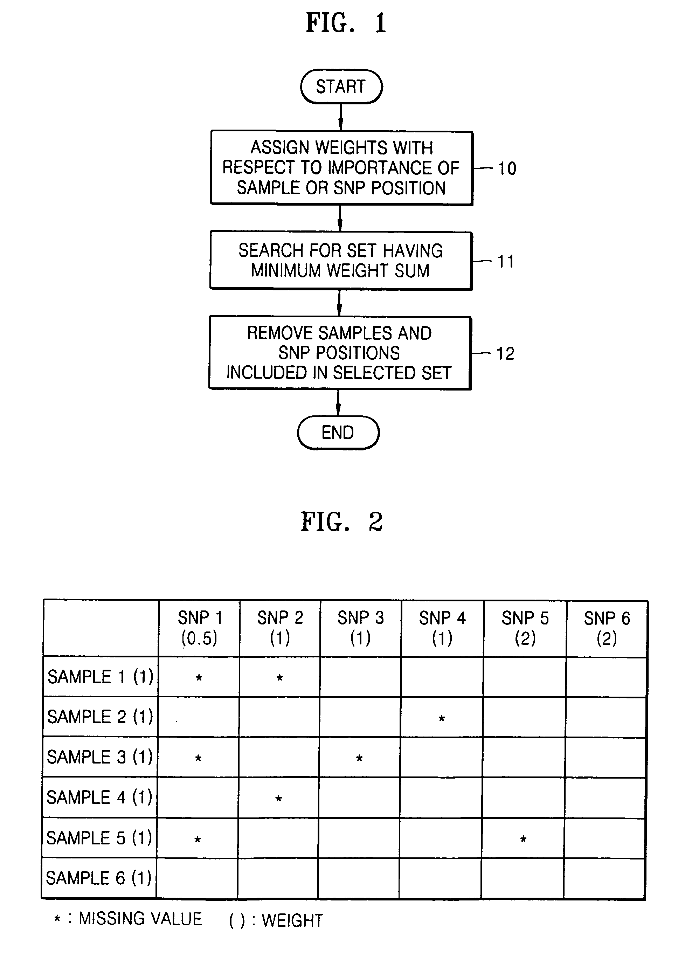

FIG.1 is a flowchart of a method for processing missing values according to an embodiment of the present invention.

Referring to FIG. 1, first, a different weight is assigned to a measured object or a measured item according to an importance of the measured object or item in step 10. In the present embodiment, the measured object is a measured sample, and the measured item is a position of a single nucleotide polymorphism (SNP). The importance of a measured sample can be determined according to an experiment subject. Also, the importance of the SNP position can also be determined by reflecting the relation with a predetermined disease.

FIG. 2 illustrates a case where an identical weight is assigned to 6 samples and different weights are assigned with respect to SNP positions. The numbers in parentheses represent weights assigned to corresponding samples or SNP positions, and '*' indicates that genetic data is missing.

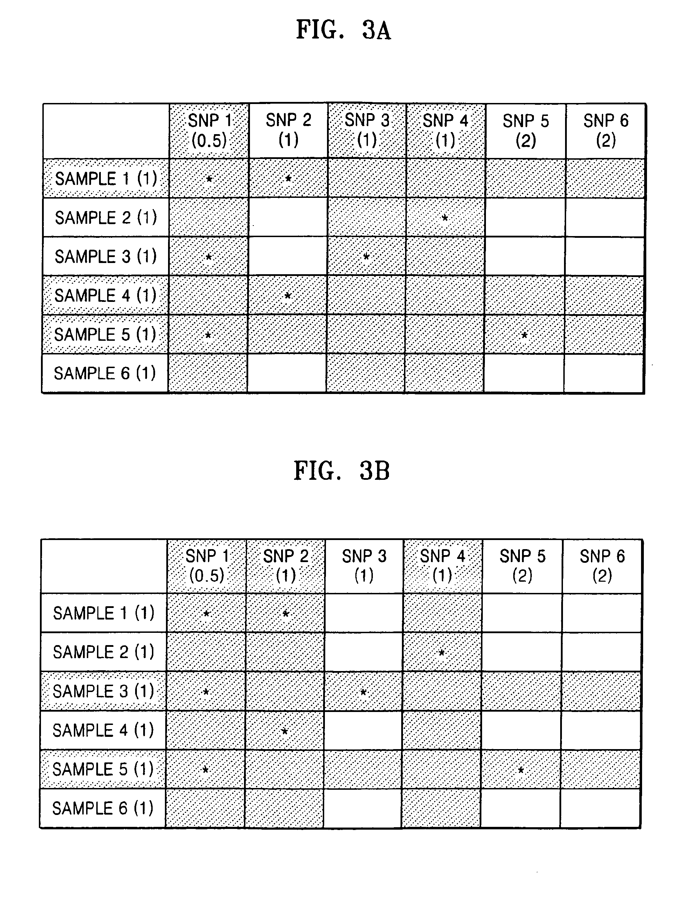

Next, among all sets of samples and SNP positions including missing values, a set having a minimum sum of weights is searched for in step 11. FIGS. 3A and 3B are showing different examples of sets of samples and SNP positions that can be removed in relation to the missing values shown in FIG. 2.

In FIG. 3A, samples 1, 4, and 5, and SNP positions 1, 3, and 4 can be removed, respectively, and at this time, a sum of weights of corresponding rows and columns is 5.5. In FIG. 3B, samples 3 and 4, and SNP positions 1, 2, and 4 can be removed and a sum of weights of corresponding rows and columns is 4.5.
Accordingly, between FIGS. 3A and 3B, the case in FIG. 3B having a smaller sum of weights is selected.

If the set of the minimum sum of weights is selected, and the samples and SNP positions included in the selected set are removed from the genetic data in step 12.

Here, the step for selecting a set of samples and SNP positions having the minimum sum of weights will now be explained in more detail. Generally, when the number of samples or SNP positions is small as in FIGS. 3A and 3B, an analyzer can manually calculate the solution by comparing a number of cases in relation to missing values. However, if the number of samples or SNP positions is too big such that it is too complex for the analyzer to manually calculate, a computer is needed to process this.

To solve this, the present embodiment adopts a bipartite graph and a method for solving a weighted vertex cover problem.

First, the analyzed data is expressed through a bipartite graph. Data shown in FIG. 2 will be explained as an example. Samples are placed as vertexes of one side of a bipartite graph and SNP positions are placed as vertexes of the other side of the bipartite graph. To each vertex, a weight of a corresponding SNP position or sample is assigned. Then, vertexes corresponding to samples and SNP positions corresponding to respective missing values are connected with edges. That is, when a missing value occurs at SNP position j in sample i, vertexes corresponding to sample i and SNP position j are connected with an edge.

The bipartite graph to which thus weights are assigned can convert to a weighted vertex cover problem. A vertex cover is a partial set of vertex sets covering all edges in the graph. The weighted vertex cover problem is to obtain a vertex cover that minimizes a sum of weights. That is, SNP positions or samples, corresponding to vertexes included in a set obtained as the solution of the weighted vertex cover problem are removed. The remaining vertexes not included in the solution form an independent set.

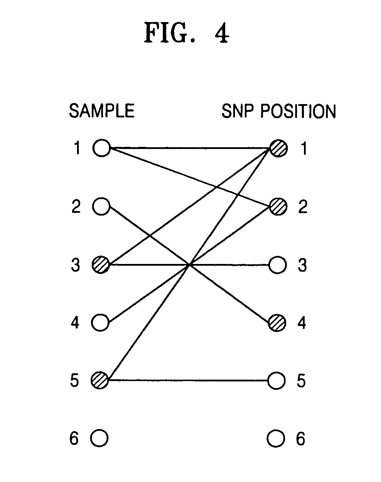

FIG. 4 illustrates a bipartite graph of data shown in FIG. 2, and among vertexes, vertexes marked by solid lines correspond to samples and SNP positions to be removed.

The vertex cover problem can be solved in a variety of methods, and Hungarian method is a leading one. The Hungarian method is disclosed in a book by Christos H. Papadimitiou and Kenneth Steiglitz, "Combinatorial optimization: Algorithms and complexity", 1982, Prentice-Hall.

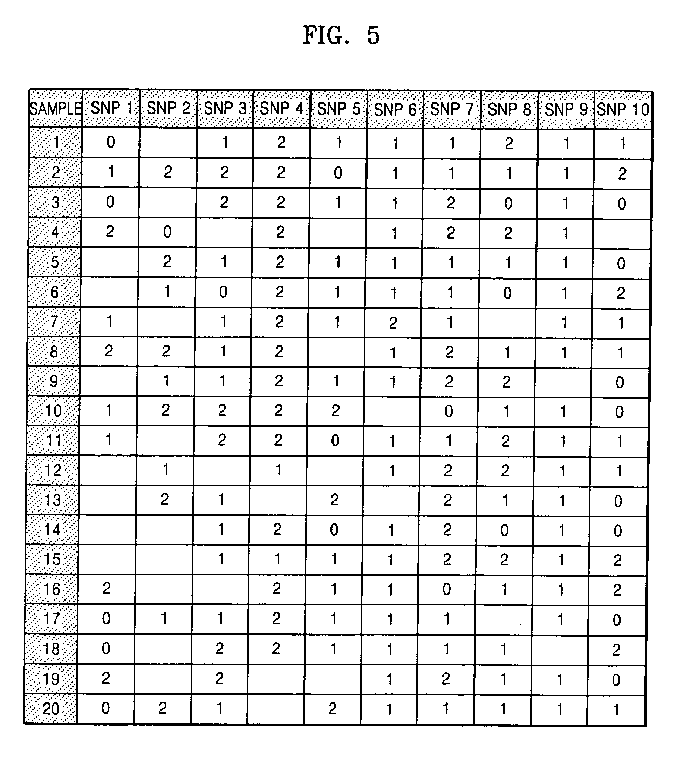

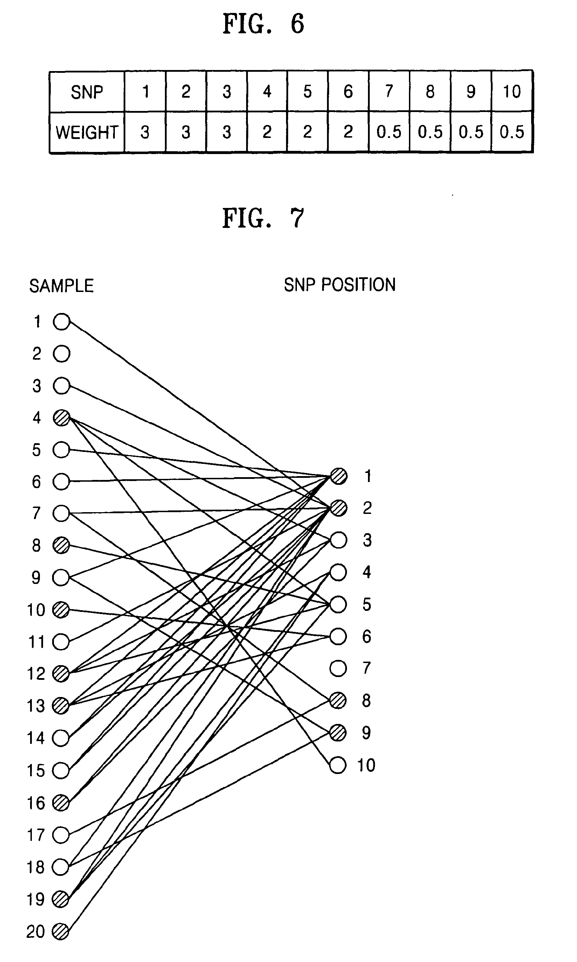

FIG. 5 illustrates an output of genetic data in which the number of SNP positions is 10, the number of samples is 20, and the number of missing values is 33. In the table, an empty cell represents a missing value, and 0, 1, and 2 are values expressing the types of nucleotide sequences, respectively, in numbers, which can be obtained from two chromosomes in one sample in each SNP position. For example, 0 denotes AA, 1 denotes AT, and 2 denotes TT. A weight for each sample is uniformly determined as 1, and a weight with respect to the SNP position is determined as shown in FIG. 6.

FIG. 7 is a bipartite graph of missing values shown in FIG. 5.

When the bipartite graph and weights are converted into a weighted vertex cover problem to solve, SNP positions 1, 2, 8, and 9, and samples 4, 8, 10, 12, 13, 16, 19, and 20 are removed such that all missing values can be removed. Also, the sum of weights of this set is 13, which is the minimum among sums of weights of sets of SNP positions and samples including all missing values.

The method for processing missing values in genetic data according to the present invention can also be embodied as computer readable codes on a computer readable recording medium. The computer readable recording medium is any data storage device that can store data which can be thereafter read by a computer system.

Examples of the computer readable recording medium include read-only memory (ROM), random-access memory (RAM), CD-ROMs, magnetic tapes, floppy disks, optical data storage devices, and carrier waves such as data transmission through the Internet. The computer readable recording medium can also be distributed over network coupled computer systems so that the computer readable code is stored and executed in a distributed fashion.

According to the present invention, in processing missing data that occurs frequently in analyzing genes, different weights are assigned with respect to the importance of a sample or a SNP position, and a set of samples and SNP positions that minimizes sum of weights is obtained and then, by removing missing data included in the set, loss of data with relatively higher importance can be minimized.

## Claims

1. A method for processing missing values in measured data including a plurality of missing values, comprising:
assigning different weights to measured objects or measured items with respect to the importance of the measured objects or measured items;
selecting a set minimizing sum of the weights, among sets of the measured objects and measured items including the missing values; and
removing measured objects and measured items included in the selected set, from the measured data.

2. The method of claim 1, wherein selecting the set minimizing the sum of the weights comprises:
generating a bipartite graph by placing the measured objects as vertexes of one side of the bipartite graph, and the measured items as vertexes of the other side, and connecting a measured object and measured item corresponding to each missing value with an edge;
assigning each vertex of the measured objects and measured items, a corresponding weight; and
selecting a set of vertexes having a minimum sum of weights of vertexes, among sets of vertexes including all edges.

3. The method of claim 1, wherein selecting the set minimizing the sum of weights comprises:
generating a bipartite graph by placing the measured objects as vertexes of one side of the bipartite graph, and the measured items as vertexes of the other side, and connecting a measured object and measured item corresponding to each missing value with an edge; and
converting the bipartite graph into a weighted vertex cover problem to solve the weighted vertex cover problem and selecting the set.

4. A method for processing missing values comprising:
assigning different weights to samples or single nucleotide polymorphism positions with respect to the importance of the samples or single nucleotide polymorphism positions in genetic data;
selecting a set minimizing a sum of weights, among sets of the samples and single nucleotide polymorphism positions including missing values; and
removing samples and single nucleotide polymorphism positions included in the selected set, from the genetic data.

5. The method of claim 4, wherein the weights assigned to the single nucleotide polymorphism position vary according to weight information including a relation of a predetermined disease with the single nucleotide polymorphism position.

6. The method of claim 4, wherein selecting the set minimizing the sum of the weights comprises:
generating a bipartite graph by placing the samples as vertexes of one side of the bipartite graph, and the single nucleotide polymorphism positions as vertexes of the other side, and connecting a sample and single nucleotide polymorphism position corresponding to each missing value with an edge;
assigning each vertex of the samples and single nucleotide polymorphism positions, a corresponding weight; and
selecting a set of vertexes having a minimum sum of weights of vertexes, among sets of vertexes including all edges.

7. The method of claim 4, wherein selecting the set minimizing the sum of the weights comprises:
generating a bipartite graph by placing the samples as vertexes of one side of the bipartite graph, and the single nucleotide polymorphism positions as vertexes of the other side, and connecting a sample and single nucleotide polymorphism position corresponding to each missing value with an edge; and
converting the bipartite graph into a weighted vertex cover problem to solve the weighted vertex cover problem and selecting the set.

8. A computer readable recording medium having embodied thereon a computer program for any one method of claims 1 through 3.

9. A computer readable recording medium having embodied thereon a computer program for any one method of claims 4 through 7.
